# EUROPEAN PATENT APPLICATION

(11) **EP 3 587 054 A1**
(43) Date of publication of application: **01.01.2020**
(21) Application number: 17897340.0
(22) Date of filing: 27.02.2017
(51) Int. Cl.: B25J 17/02, A61B 1/018, A61B 17/94, B25J 17/00

(54) **MANIPULATOR**

(71) Applicant: Asahi Intecc Co., Ltd., Seto-shi, Aichi 489-0071 (JP)
(72) Inventor: YAMAGUCHI, Shinji, Tokyo (JP)
(74) Representative: Viering, Jentschura & Partner mbB Patent- und Rechtsanwälte
(86) International application number: PCT/JP2017/007489
(87) International publication number: WO 2018/154780

(57) **Abstract**

A manipulator comprising: an operation portion, wherein forceps are opened and closed, when a first forceps pulley of a first forceps piece and a second forceps pulley of a second forceps piece rotate in opposite directions to each other, a direction of the forceps is changed when both pulleys rotate in the same direction, and a direction of the forceps can be changed about a P axis (pitch axis); a first driving portion comprising a left driving pulley and a right driving pulley slidably movable along a Z axis (main axis) direction; and an endless loop-shaped wire rope, wherein in the loop-shaped wire rope, a first engaging body, a second engaging body, a third engaging body and a fourth engaging body are provided one after another on its loop-shaped wire rope main body, and the loop-shaped wire rope is arranged to engage the first engaging body to the first forceps pulley, the second engaging body to the second forceps pulley, the third engaging body to a left driving pulley, and the fourth engaging body to a right driving pulley.

## Description

### FIELD OF THE INVENTION

The present invention relates to a rope-driven manipulator for remote control of an operation portion such as forceps via a wire rope (hereinafter referred to as "rope" for short).

### BACKGROUND ARTS

A rope-driven manipulator has a multi-articulated structure for individually rotating operation portions such as forceps provided on its tip around two or three axes perpendicular to each other. For example, forceps for surgery as operation portions perform an opening and closing movement for rotating a pair of forceps pieces in different directions around a Y-axis (or yaw axis), a grip direction changing movement for rotating the pair of forceps pieces in an identical direction around the Y-axis in order to change a direction of a grip portion, and a swinging movement for rotating a holding member for holding the grip portion around a P-axis (or pitch axis) perpendicular to the Y-axis (Patent Literature 1).

A rope-driving portion of the rope-driven manipulator is configured to stretch loop-shaped wire ropes (hereinafter referred to as "loop-shaped ropes" for short) for independently driving each blade are hooked between a blade pulley and a blade driving motor pulley, for an opening and closing movement and a grip direction changing movement, as described above. On the other hand, the rope-driving portion for swinging movement has loop-shaped ropes hooked between a pulley for swinging driving and a swinging driving motor pulley. Tension detecting means are provided on each loop-shaped wire rope to detect a tension of the loop-shaped ropes.

### CITATION LIST

### PATENT LITERATURE

Patent Literature 1: JP Patent Application Publication No. 2004-122286

### SUMMARY OF THE INVENTION

### PROBLEMS TO BE SOLVED

In conventional rope-driven manipulators, a rope-driving portion is provided with a plurality of loop-shaped ropes, so that there is a need for tension adjustment for each loop-shaped rope.

On the other hand, it is conceivable to enhance degrees of freedom by rotating each of operation portions disposed as spaced apart on a front and rear portions and a corresponding one of drive source portions composed of two blade drive motors and a swinging driving motor relative to each other about an axial center of a longitudinal axis. In this case, when the operation portion is rotated relative to the corresponding drive source portion about the longitudinal axis, a loop-shaped rope is twisted to change the tension, so that no smooth operation and precise operation of the operation portions can be achieved.

An object of the invention is to provide a manipulator capable of simplifying a tension adjustment of a loop-shaped rope.

A further object of the invention is, in addition to the above-mentioned object, to provide a manipulator capable of maintaining a tension of a loop-shaped rope to an appropriate value, even if a loop-shaped rope is twisted.

A manipulator to achieve a first object of the invention includes: a manipulator main body extending along a main axis direction; an operation portion in which a first operation piece having a first rotating body and a second operation piece having a second rotating body are rotatably provided on a first axis along a axis direction, the operation portion being attached to a front edge portion of the manipulator main body so as to be rotatable about a second axis perpendicular to a main axis, along with rotation of the first rotating body and the second rotating body in mutually different rotation directions, an opening and closing operation of the first operation piece and the second operation piece thereby being performed; a left driving portion provided with a third rotating body and a right driving portion provided with a fourth rotating body, both of the driving portions being disposed on the right and left of a rear end portion of the manipulator main body and movable along the main axis direction; a loop-shaped wire rope formed from an endless loop-shaped wire rope main body folded by each of the first to the fourth rotating bodies at four fixing spots sequentially from a first fixing spot, a second, a third and a fourth fixing spots at intervals from one another, the first fixing spot being fixed to the first rotating body, the second fixing spot being fixed to the second rotating body, the third fixing spot being fixed to the third rotating body, and the fourth fixing spot being fixed to the fourth rotating body; a fifth rotating body provided on a rear end portion of the loop-shaped wire rope main body with a position of the fifth rotating body being variable along the main axis direction, the fifth rotating body being rotatable; and a non-loop-shaped wire rope folded at the fifth rotating body, one end of the wire rope being fixed to the left driving portion, the other end to the right driving portion, and a central fixing spot of the wire rope to the fifth rotating body.

A manipulator to achieve the other object of the invention includes: a manipulator main body extending along a main axis direction; an operation portion in which a first operation piece having a first rotating body and a second operation piece having a second rotating body are rotatably provided on a first axis along a axis direction, the operation portion being attached to a front edge portion of the manipulator main body so as to be rotatable about a second axis perpendicular to a main axis, along with rotation of the first rotating body and the second rotating body in mutually different rotation directions an opening and closing operation of the first operation piece and the second operation piece thereby being performed; a first base portion disposed on a rear end portion of the manipulator main body to be relatively rotatable to the manipulator main body; a second base portion disposed rearward of the manipulator main body with a fixed position of the second base portion to the first base portion being variable, and being relatively rotatable to the manipulator main body; a left driving portion provided with a third rotating body and a right driving portion provided with a fourth rotating body, both of the driving portions being disposed on the right and left of a rear end portion of the first base and movable along the main axis direction; a loop-shaped wire rope formed from an endless loop-shaped wire rope main body folded by each of the first to the fourth rotating bodies at four fixing spots sequentially from a first fixing spot, a second, a third and a fourth fixing spots at intervals from one another, the first fixing spot being fixed to the first rotating body, the second fixing spot being fixed to the second rotating body, the third fixing spot being fixed to the third rotating body, and the fourth fixing spot being fixed to the fourth rotating body; a fifth rotating body rotatably provided on the second base portion; a non-loop-shaped wire rope folded at the fifth rotating body, wherein one end of the wire rope is fixed to the left driving portion, the other end is fixed to the right driving portion, and a central fixing spot of the wire rope is fixed to its fifth rotating body; and a cam portion fixed to the manipulator main body to make the manipulator main body movable along the main axis direction following a rotation about the main axis.

According to a first aspect of the invention, a single loop-shaped wire rope enables opening and closing movements of a pair of operation pieces, as well as direction changing movements about a first axis and a second axis. In this manner, it is possible to simplify a tension adjustment work of the loop-shaped wire rope. Moreover, a movement targeted each time does not affect other movements, in order to achieve a high-speed response to movement.

According to a second aspect of the invention, a rotation of a manipulator main body allows the manipulator main body to move along a main axis direction even if a loop-shaped wire rope is twisted, in order to assure a maintenance of an appropriate tension. In this manner, it is possible to achieve an operation feeling with no discomfort.

### BRIEF DESCRIPTION OF THE DRAWINGS

FIG. 1 shows a perspective view of appearance of a manipulator according to a first embodiment of the invention;
FIG. 2 is an exploded perspective view of a manipulator as shown in FIG. 1;
FIG. 3A is a perspective view illustrating an arranged state in a manipulator as shown in FIG. 1, in which a loop-shaped rope is hooked on pulleys;
FIG. 3B is a plan view of a loop-shaped rope;
FIG. 3C is a plan view of a non-loop-shaped rope;
FIG. 4 is a perspective view of appearance of a tip of a manipulator as shown in FIG. 1;
FIG. 5 is a perspective view of appearance of a drive portion of a manipulator as shown in FIG. 1;
FIG. 6 shows a perspective view of appearance of a manipulator according to a second embodiment of the invention;
FIG. 7 is an exploded perspective view of a manipulator as shown in FIG. 6;
FIG. 8 is a perspective view illustrating an arranged state in a manipulator as shown in FIG. 6, in which loop-shaped rope is hooked on pulleys;
FIG. 9 is a perspective view illustrating an arranged state in a manipulator as shown in FIG. 8 in which a loop-shaped rope and a non-loop-shaped rope are hooked on pulleys;
FIG. 10 is an exploded perspective view a second and a third driving portions of a manipulator as shown in FIG. 6, as seen from below;
FIG. 11A is an exploded perspective view a second and a third driving portions of a manipulator as shown in FIG. 6, as seen from above;
FIG. 11B is a view illustrating a cam surface of a cylindrical cam portion of a third driving portion as shown in FIG. 11A; and
FIG. 12 is a view illustrating a state, in which a supporting pipe 1 of a manipulator as shown in FIG. 8 is rotated 90 degrees.

### DETAILED DESCRIPTION OF THE INVENTION

In the following, the invention is explained in detail, based on embodiments shown in drawings

### Embodiment 1

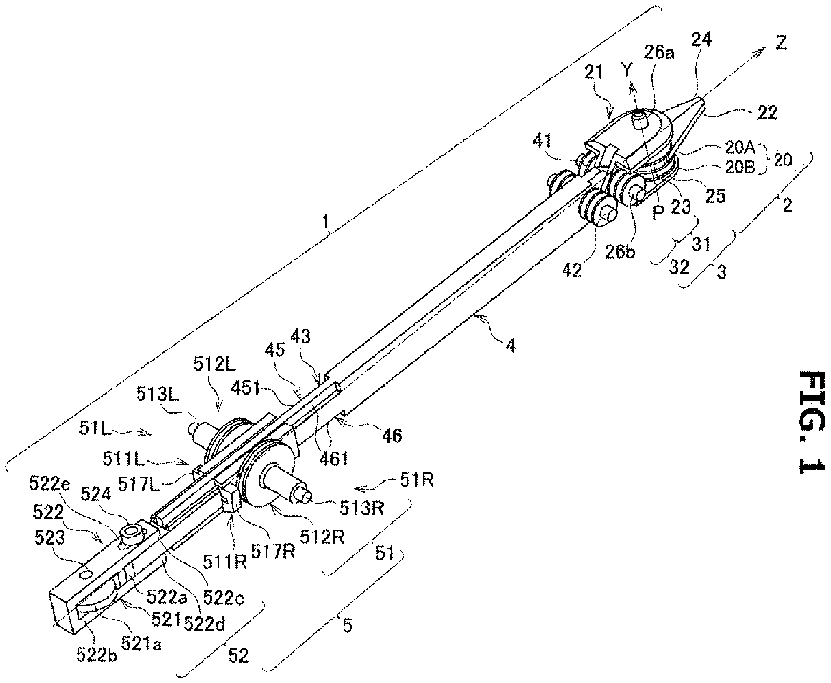

FIGS. 1 to 5 show Embodiment 1 of the invention.

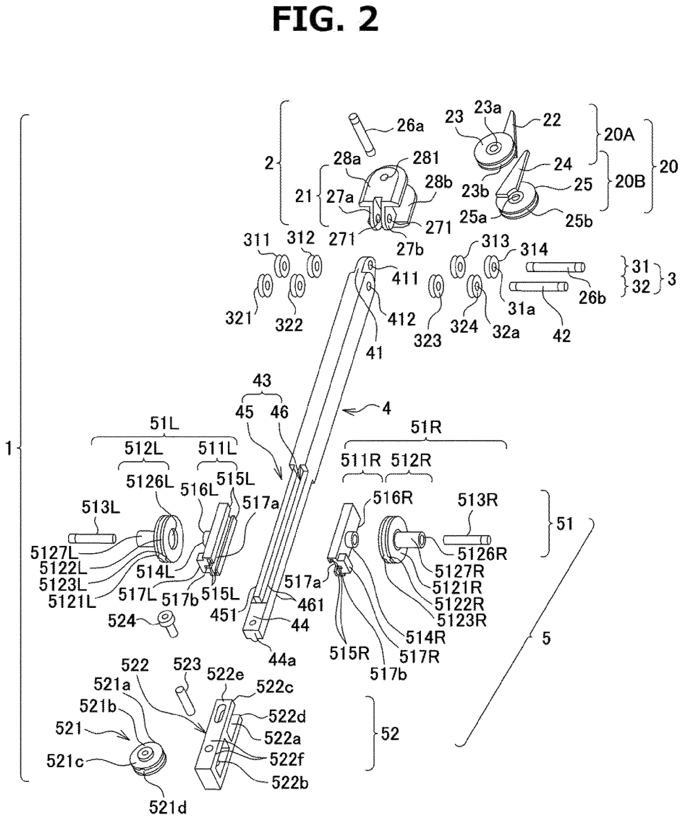

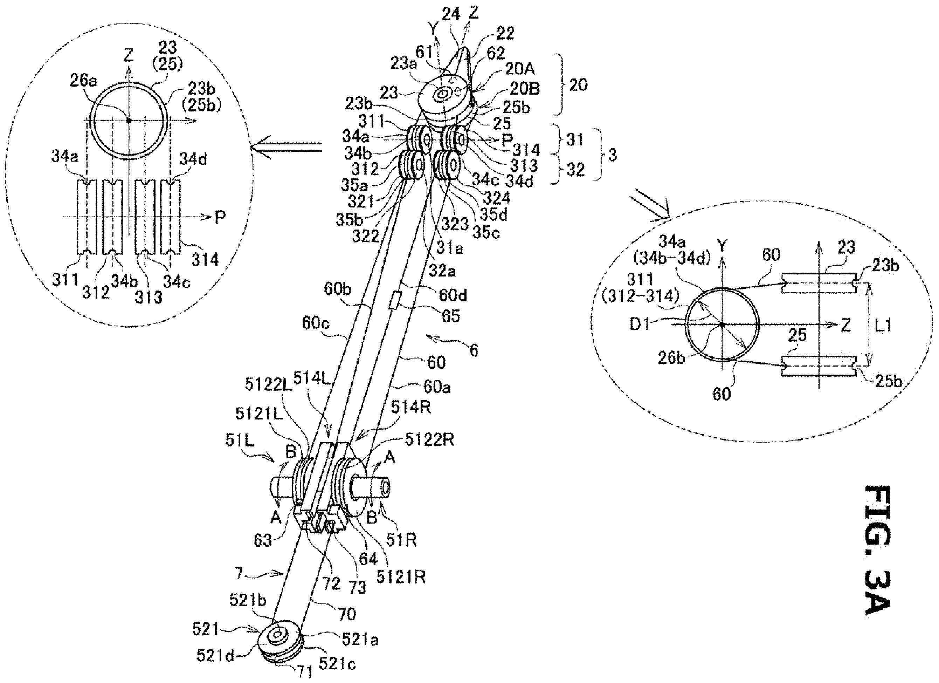

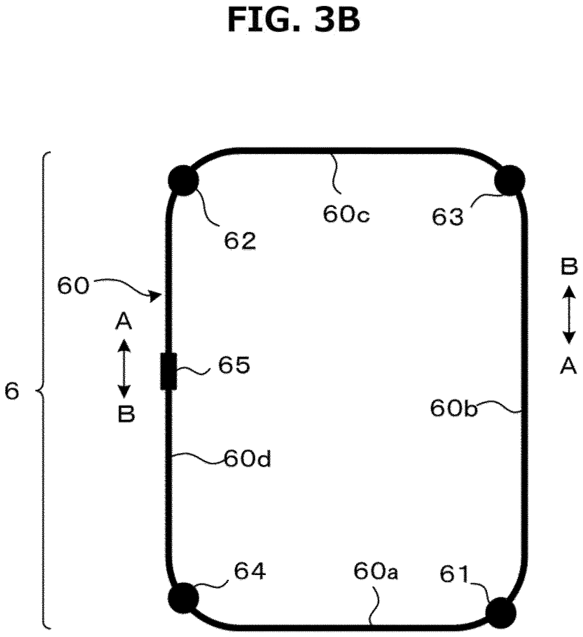

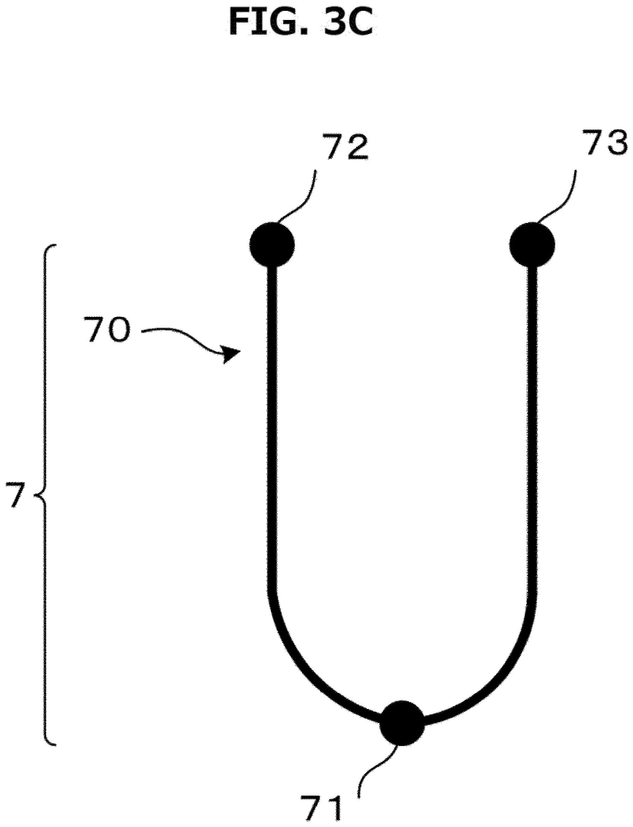

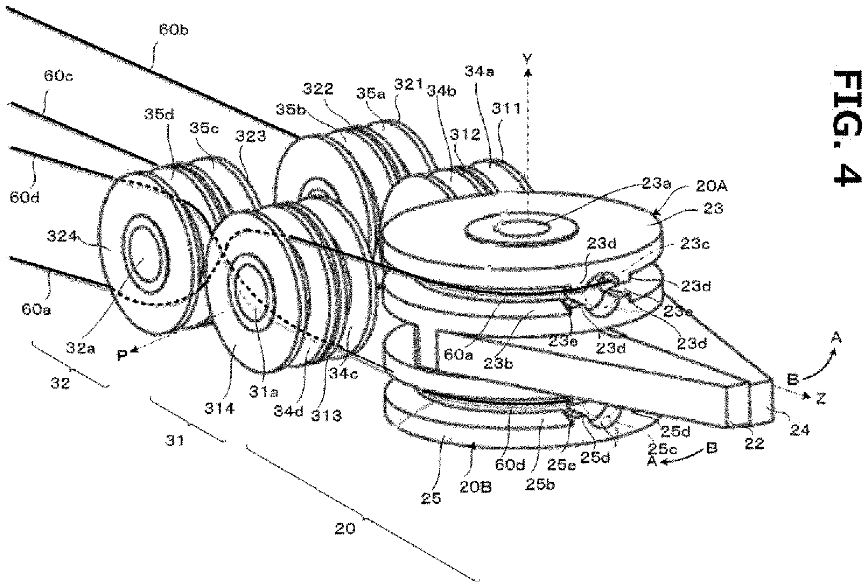

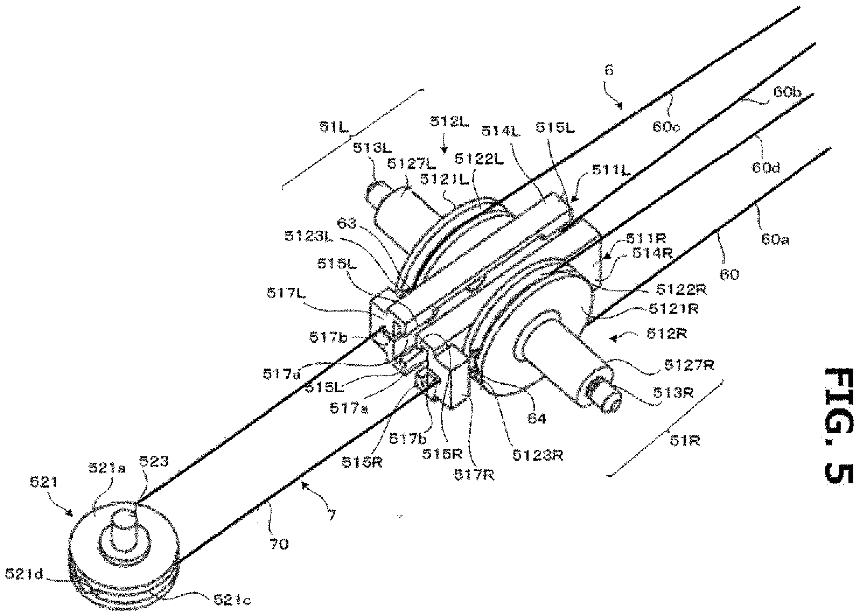

FIG. 1 shows a perspective view of appearance of a manipulator according to a first embodiment of the invention; FIG. 2 is an exploded perspective view of a manipulator as shown in FIG. 1; FIG. 3A is a perspective view illustrating an arranged state in a manipulator as shown in FIG. 1, in which a loop-shaped rope is hooked on pulleys; FIG. 3B is a plan view of a loop-shaped rope; FIG. 3C is a plan view of a rope with ends; FIG. 4 is a perspective view of appearance of a tip of a manipulator as shown in FIG. 1; and FIG. 5 is a perspective view of appearance of a drive portion of a manipulator as shown in FIG. 1.

### Overall Structure of Manipulator 1

A manipulator 1 includes an operation portion 2, a tip-side pulley portion 3 being a tip-side rotating body portion, an arm portion 4 being a manipulator main body, a driving portion 5, a loop-shaped wire rope (referred to as "loop-shaped rope" for short) 6, and non-loop-shaped wire rope with ends (hereinafter referred to as "non-loop-shaped rope" for short) 7. Here, in the manipulator 1, three axes perpendicular to each other are referred to as a Z-axis being a main axis, a Y-axis (or yaw axis) being a first axis, and a P-axis (or pitch axis) being a second axis. In the meantime, the Y-axis crosses the Z-axis at right angles at a neutral position shown in FIG. 1 in the operation portion 2. Furthermore, a P-axis direction is also referred to as a right and left direction, a Z-axis direction as a forward and backward direction, and a Y-axis direction as an upward and downward direction.

### Structure of Operation Portion 2

In this embodiment, an operation portion 2 includes a forceps 20 being an operating body and a holder 21 for rotatably holding the forceps 20 about a Y-axis.

Forceps 20 includes a first forceps piece 20A and a second forceps piece 20B, both being operation pieces. In the first forceps piece 20A, a first forceps pulley 23 being a first rotating body is integrally formed on a base end portion of a first forceps piece main body 22, while in the second forceps piece 20B, a second forceps pulley 25 being a second rotating body is integrally formed on a base end portion of a second forceps piece main body 24. A first axial hole 23a and a second axial hole 25a are formed on respective central portions of the first forceps pulley 23 and the second forceps pulley 25. The first forceps piece main body 22 is formed on one side on a lower surface of the first forceps pulley 23 to avoid the first axial hole 23a. The second forceps piece main body 24 is formed on one side on an upper surface of the second forceps pulley 25 to avoid the second axial hole 25a.

A first rope groove 23b and a second rope groove 25b are formed on respective outer circumferences of a first forceps pulley 23 and a second forceps pulley 25. A loop-shaped rope main body 60 of a loop-shaped rope 6 as shown in FIG. 3B to be described below is hooked on each of the rope grooves 23b and 25b. As shown in FIG. 4, a first engaging hole 23c and a second engaging hole 25c are formed on respective spots of the first rope groove 23b and the second rope groove 25b. As shown in FIG. 3A, a first engaging body 61 and a second engaging body 62 both in the shape of ball which are caulked and thus fixed to the rope main body 60 are engaged with the first engaging hole 23c and the second engaging hole 25c, wherein the first forceps pulley 23 and the second forceps pulley 25 are rotated by a pulling action of the rope main body 60 without slipping relative to the rope main body 60. The first forceps pulley 23 and the second forceps pulley 25 only need to be fixed to the rope main body 60 without slipping in a tangential direction, and approaches for engaging are not limited to that using engaging bodies and engaging holes. This also applies to a third engaging body 63, a fourth engaging body 64, a left driving pulley 512L and a right driving pulley 512R to be described below.

Communication grooves 23e, 25e are formed on a partition wall 23d separating a first engaging hole 23c from a first rope groove 23b and a partition wall 25d separating a second engaging hole 25c from a second rope groove 25b. A communication groove 23e and a communication groove 25e are formed in such a groove width that allows a rope main body 60 to be fitted into each of them.

A first forceps piece 20A and a second forceps piece 20B are formed to have an identical structure. As shown in FIGS. 2 and 4, forceps 20 have a structure in which inner surface sides (the sides provided with a first forceps piece main body 22 and a second forceps piece main body 24) of a first forceps pulley 23 and of a second forceps pulley 25 to face each other and aligning axial centers of a first axial hole 23a and a second axial hole 25a to overlap each other. FIG. 4 shows a state in which the first forceps piece 20A and the second forceps piece 20B are closed, and centers of a first axial hole 23a, a second axial hole 25a, a first engaging hole 23c and a second engaging hole 25c are located on an extension line of a gripping surface on which the first forceps piece 20A and the second forceps piece 20B abut against each other.

A holder 21 pivotally supports forceps 20 by a Y supporting axis 26a being a second supporting axis such that they are rotatable about a Y axis. Still further, the holder 21 is pivotally supported on a tip of an arm portion 4 by a P supporting axis 26b being a first supporting axis to be rotatable about a P axis.

A holder 21 includes supporting brackets 27a, 27b on the right and left facing each other with a predetermined interval in a P axis direction, and a pair of supporting plates 28a, 28b on the right and left facing each other with a predetermined interval along a Y axis direction. The pair of supporting plates 28a, 28b extend along a Z axis direction from both end portions of a left supporting bracket 27a and a right supporting bracket 27b along the Y axis direction.

An inserting tip portion 41 of an arm portion 4 is inserted between a left supporting bracket 27a and a right supporting bracket 27b without a backlash. Axial holes 271 into which a P supporting axis 26b is inserted to be freely rotatable are formed respectively on the left supporting bracket 27a and on the right supporting bracket 27b. Still further, a front axial hole 411 into which a P supporting axis 26b is inserted to be freely rotatable is formed respectively on the inserting tip portion 41. The inserting tip portion 41 is inserted between the left supporting bracket 27a and the right supporting bracket 27b, and the P supporting axis 26b is inserted into each of the axial holes 271 and the front axial hole 411, wherein all the axial holes are aligned. Therefore, the holder 21 is attached to the arm portion 4 to be freely rotatable about a P axis. In the meantime, retainer members (not shown) are attached to both end portions of the P supporting axis 26b, in order to prevent a fall of the P supporting axis 26b.

Axial holes 281 (of which an axial hole 281 on a supporting plate 28b is not shown) are formed on a pair of supporting plates 28a, 28b of a holder 21. Forceps 20 are disposed between the supporting plate 28a and the supporting plate 28b without a backlash. A Y supporting axis 26a runs from an axial hole 281 on the supporting plate 28a via a first axial hole 23a of a first forceps piece 20A and a second axial hole 23b of a second forceps piece 20B, in order to pass through the axial hole 281 on the supporting plate 28b. In the meantime, retainer members (not shown) are attached to both end portions of the Y supporting axis 26a, in order to prevent a fall of the Y supporting axis 26a. Namely, the first axial hole 23a and the second axial hole 23b are pivotally supported to the Y supporting axis 26a to be freely rotatable. Therefore, the first forceps piece 20A and the second forceps piece 20B are held to the holder 21 to be individually freely rotatable about a Y axis.

### Structure of Tip-Side Pulley Portion 3

A tip-side pulley portion 3 includes a front row pulley portion 31 and a rear row pulley portion 32. The front row pulley portion 31 and the rear row pulley portion 32 are disposed on the front and rear along a Z axis direction. A pulley distance between the front row pulley portion 31 and the rear row pulley portion 32 includes a gap for allowing at least a rope main body 60.

A front row pulley portion 31 includes a first outer front pulley 311, a first inner front pulley 312, a second inner front pulley 313 and a second outer front pulley 314. A rear row pulley portion 32 includes a first outer rear pulley 321, a first inner rear pulley 322, a second inner rear pulley 323 and a second outer rear pulley 324. Each pulley of the front row pulley portion 31 and the rear row pulley portion 32 is formed to have an identical dimension.

A front pulley axial hole 31a is formed on each of a first outer front pulley 311, a first inner front pulley 312, a second inner front pulley 313 and a second outer front pulley 314. A rear pulley axial hole 32b is formed on each of a first outer rear pulley 321, a first inner rear pulley 322, a second inner rear pulley 323 and a second outer rear pulley 324.

Front rope grooves 34a to 34d on which a loop-shaped rope 6 is hooked are formed on respective outer circumferential portions of a first outer front pulley 311, a first inner front pulley 312, a second inner front pulley 313 and a second outer front pulley 314. Rear rope grooves 35a to 35d on which a rope main body 60 is hooked are formed on respective outer circumferential portions of a first outer rear pulley 321, a first inner rear pulley 322, a second inner rear pulley 323 and a second outer rear pulley 324.

A first outer front pulley 311 and a first inner front pulley 312 are disposed outside a left supporting bracket 27a, while a second inner front pulley 313 and a second outer front pulley 314 outside a right supporting bracket 27b. A P supporting axis 26b rotatably passes through each of front pulley axial holes 31a of the first outer front pulley 311, the first inner front pulley 312, the second inner front pulley 313 and the second outer front pulley 314. The first outer front pulley 311 is disposed outside the first inner front pulley 312, while a second outer front pulley 314 outside the second inner front pulley 313.

In this embodiment, an intermediate position between a first rope groove 23b of a first forceps piece 20A and a second rope groove 25b of a second forceps piece 20B in a Y axis direction coincides with an axial center position of the P supporting axis 26b.

Where an outer diameter of each of front rope grooves 34a to 34d of each pulley (311 to 314) constituting a front row pulley portion 31 is defined as D1, and a distance between a first rope groove 23b of a first forceps piece 20A and a second rope groove 25b of a first forceps piece 20B in a Y axis direction as L1, D1 is set to be smaller than L1. Namely, a rope main body 60 hooked between a first rope groove 23b and a second rope groove 25b on one hand and the front rope grooves 34a to 34d of each pulley of the front row pulley portion 31 on the other is arranged to be inclined relative to each pulley of the front row pulley portion 31 toward a P supporting axis 26b.

Still further, a front rope groove 34a of a first outer front pulley 311 and a front rope groove 34d of a second outer front pulley 314 are located outside in a P axis direction of respective outermost end portions in the P axis direction of a first rope groove 23b (a second rope groove 25b), while a front rope groove 34a of a first outer front pulley 312 and a front rope groove 34d of a second outer front pulley 313 are inside.

A first outer rear pulley 321, a first inner rear pulley 322, a second inner rear pulley 323 and a second outer rear pulley 324 of a rear row pulley portion 32 have their respective axial holes 32a pivotally supported by a rear supporting axis 42 to be freely rotatable. The rear supporting axes 42 is pivotally supported by a rear axial hole 412 of an arm portion 4 to be freely rotatable. The rear axial hole 412 is formed rearward from a front axial hole 411 of the arm portion 4. The rear axial hole 412 and the front axial hole 411 are provided at the same height in an upward and downward direction.

A first inner rear pulley 321 and a first inner rear pulley 322 are disposed on the left of an arm portion 4, while a second inner rear pulley 323 and a second outer rear pulley 324 on the right of the arm portion 4. Still further, the first outer rear pulley 321 and the first inner rear pulley 322 are disposed right behind a first outer front pulley 311 and a first inner front pulley 312. The second inner rear pulley 323 and the second outer rear pulley 324 are disposed right behind a second inner front pulley 313 and a second outer front pulley 314. In the meantime, retainer members (not shown) are provided outside the first outer rear pulley 321 and the second outer rear pulley 324 on both axial end portions of a rear supporting axis 42, in order to prevent each pulley of a rear pulley portion 32.

### Structure of Arm Portion 4

An arm portion 4 is formed in the shape of elongate prismatic column, wherein an inserting tip portion 41 is formed on its tip, a driving portion attaching portion 43 on its rear end portion, and a screw hole portion 44 formed along a Y axis direction is provided rearward from the driving portion attaching portion 43.

A driving portion attaching portion 43 includes a left guide rail portion 45 formed on a left lateral portion of an arm portion 4 and a right guide rail portion 46 formed on its right lateral portion. On the left guide rail portion 45, a left rail groove 451 extending in a forward and backward direction is formed above and below. On the right guide rail portion 46, a right rail groove 461 extending in a forward and backward direction is formed in an upward and downward direction. The left rail groove 451 and the right rail groove 461 are formed symmetrically on the right and left, wherein their traverse sections above and below are formed in a substantially concave shape.

### Structure of Drive Portion 5

A driving portion 5 includes a first driving portion 51 provided on a driving portion attaching portion 43 and a second driving portion 52 fixed to a screw hole portion 44.

A first driving portion 51 includes a left driving portion 51L provided on a left guide rail portion 45 and a right driving portion 51R provided on a right guide rail portion 46, wherein the left driving portion 51L and the right driving portion 51R are formed symmetrically on the right and left.

A left driving portion 51L includes a left slider 511L, a left driving pulley 512L being a third rotating body and a motor axis 513L of a left driving motor (not shown). In the left slider 511L, engaging claw portions 515L for engaging with a left rail groove 451 above and below are formed at four corners on an inner side (right-hand side) of its left slider main body 514L. A pulley supporting axis 516L for pivotally supporting the left driving pulley 512L is formed along a P-axis direction on an outer side (left-hand side) of the left slider main body 514L. The left slider 511L is held on an arm portion 4 to be movable in a forward and backward direction of an arm portion 4 but non-movable in the P-axis direction and a Y-axis direction, by engaging four engaging claw portions 515L opposite one another in the forward and backward direction and above and below with a left rail groove 451 above and below.

A left rope fixing piece 517L is along a P-axis direction on a rear end portion outside a left slider main body 514L. The left rope fixing piece 517L includes an opening portion 517a for passing a ball-shaped left terminal portion 72 provided on a left end of a wire rope main body (hereinafter referred to as rope main body in short) 70 of a non-loop-shaped wire rope 7 to be described below and a slit 517b in communication with the opening portion 517a. The slit 517b is formed in a slit width to pass the rope main body 70 but not to pass the left terminal portion 72. The non-loop-shaped wire rope 7 is set to pass the opening portion 517a such that the left terminal portion 72 is disposed on the front side of the left rope fixing piece 517L, and to pass the rope main body 70 through the slit 517b. Therefore, the left terminal portion 72 is caught by the slit 517b, and when the rope main body 70 is pulled backward, a left slider 511L is guided backward toward a left guide rail portion 45 to slide.

In a left driving pulley 512L, a left rope groove 5122L about which a rope main body 60 is hooked is provided on an outer circumference of a left pulley main body 5121L. A third engaging hole 5123L with which a third engaging body 63 provided on the rope main body 60 of a loop-shaped rope 6 is engaged is formed on a spot in a circumferential direction of the left rope groove 5122L.

A left bearing hole 5126L is formed on the same axial center as an axial center of a left pulley main body 5121L inside (on the right of) the left pulley main body 5121L. The left bearing hole 5126L is pivotally supported by a pulley supporting axis 516L in a rotatable manner. Moreover, a hollow-shaped left pulley driving axis 5127L extending outward in a P-axis direction is formed on the same axial center as the left bearing hole 5126L outside (on the left of) the left pulley main body 5121L. A motor axis 513L of a left driving motor is inserted into a hollow portion of left pulley driving axis 5127L and fixed thereto to be non-rotatable about an axis. The above-mentioned left driving motor (not shown) is attached to an attaching frame (not shown) fixed to a left slider main body 514L.

Therefore, a left driving portion 51L is guided together with a left slider 511L by a left guide rail portion 45 of an arm portion 4 to be slidable in a forward and backward direction.

Next, a right driving portion 51R includes a right slider 511R, a right driving pulley 512R being a fourth rotating body and a motor axis 513R of a left driving motor (not shown). As these members constituting the right driving portion 51R includes identical parts as in a left driving portion 51L, the identical parts or elements have identical reference numerals, but with ending R and "left" at the head of element name being replaced by "right", and without description on their structure.

On a right slider 511R, engaging claw portions 515R for engaging with a right rail groove 461 above and below are formed at four corners on an inner side (on the left) of its right slider main body 514R. A right bearing hole 5126R (not shown) of a right driving pulley 512R is pivotally supported by a pulley supporting axis 516R formed outside (on the right of) a right slider main body 514R.

In a right rope fixing piece 517R of a right slider main body 514R, a right terminal portion 73 is caught by the slit 517b, and when the rope main body 70 is pulled backward, a right slider 511R is guided backward toward a right guide rail portion 46 to slide.

In a right driving pulley 512R, a fourth engaging body 64 of a loop-shaped rope 6 is engaged with a fourth engaging hole 5123R of a right rope groove 5122R of a right pulley main body 5121R.

A motor axis 513R of a right driving motor is inserted into a hollow portion of a right pulley driving axis 5127R and fixed thereto to be non-rotatable about an axis. The above-mentioned right driving motor (not shown) is attached to an attaching frame (not shown) fixed to a right slider main body 514R.

Therefore, a right driving portion 51R is guided together with a right slider 511R by a right guide rail portion 46 of an arm portion 4 to be slidable in a forward and backward direction.

As shown in FIG. 3B, an endless loop-shaped rope 6 includes a wire rope main body (hereinafter referred to as rope main body in short) 60. Both ends of a wire rope of a predetermined length are caulked by a closing terminal portion 65 and fixed to each other to be formed in the shape of loop. Four fixing spots for fixing a first forceps pulley 23, a second forceps pulley 25, a left driving pulley 512L and a right driving pulley 512R at respective single spots are provided on the rope main body 60, wherein in this embodiment the latter includes as fixing spots a first engaging body 61, a second engaging body 62, a third engaging body 63 and a fourth engaging body 64, respectively attached to the rope main body 60 at intervals. In the meantime, engaging bodies are used as fixing spots, but the rope main body can be also fixed by adhesive. Starting from the closing terminal portion 65, the second engaging body 62, the third engaging body 63, the first engaging body 61 and the fourth engaging body 64 are attached, as arranged in a clockwise direction. On the rope main body 60, a rope portion between the fourth engaging body 64 and the first engaging body 61 is defined as a first rope portion 60a, a rope portion between the first engaging body 61 and the third engaging body 63 - as a second rope portion 60b, a rope portion between the third engaging body 63 and the second engaging body 62 - as a third rope portion 60c, a rope portion between the second engaging body 62 and the fourth engaging body 64 - as a fourth rope portion 60d.

Next, in reference to FIGS. 3A, 3B, 4 and 5, reference is made to an arranged state where a loop-shaped rope 6 is hooked on a first forceps pulley 23, a second forceps pulley 25, a first outer front pulley 311, the first inner front pulley 312, the second inner front pulley 313 and the second outer front pulley 314, a first outer rear pulley 321, a first inner rear pulley 322, a second inner rear pulley 323, a second outer rear pulley 324, a left driving pulley 512L and a right driving pulley 512R.

Reference is made to an example of an arranging route where a rope main body 60 starts from a first engaging body 61, and returns to the first engaging body 61. The first engaging body 61 is engaged with a first engaging hole 23c of a first forceps pulley 23, and a second rope portion 60b extends from a first rope groove 23b of the first forceps pulley 23 toward an upper end side of a first outer front pulley 311. The second rope portion 60b passes through a front rope groove 34a of a first outer front pulley 311, and extends toward a lower end side of a first outer rear pulley 321 just behind the first outer front pulley. The second rope portion 60b is guided from an upper end side of the front rope groove 34a of the first outer front pulley 311 toward the lower end side of a rear rope groove 35a of the first outer rear pulley 321 to be hooked on the both rope grooves in the shape of S.

As shown in FIG. 5, a second rope portion 60b, as passing through a rear rope groove 35a of the first outer rear pulley 321, is further guided toward a lower end side of a left driving pulley 512L and hooked on a left rope groove 5122L. A third engaging body 63 is engaged with a third engaging hole 5123L. A third rope portion 60c extending from the third engaging body 63 is hooked on a left rope groove 5122L of the left driving pulley 512L, and guided toward an upper end side of a first inner rear pulley 322 further forward to be hooked on a rear rope groove 35b. Here, a second rope portion 60b and the third rope portion 60c do cross but have distance in an upward and downward direction and an inward and outward direction, so they do not have contact with each other.

A third rope portion 60c guided toward an upper end side of a second inner rear pulley 322 is further guided toward a lower end side of a first inner front pulley 312 to be hooked between a rear rope groove 35b and a front rope groove 34b in the shape of S character. Still further, the third rope portion 60c is hooked on a second rope groove 25b of a second forceps pulley 25 from the lower end side of the first inner front pulley 312. A second engaging body 62 engages a second engaging hole 25c.

A fourth rope portion 60d extending from the second engaging body 62 is guided from a second rope groove 25b toward a lower side of a second inner front pulley 313 to be hooked on a front rope groove 34c. A fourth rope portion 60d guided toward a lower end side of a second inner front pulley 313 is further guided toward an upper end side of a second inner rear pulley 323 to be hooked between a front rope groove 34c and a front rope groove 35c in the shape of S character. Still further, the fourth rope portion 60d is guided from an upper end side of a second inner rear pulley 323 toward an upper end side of a right driving pulley 512R to be hooked on a right rope groove 5122R. A fourth engaging body 64 is engaged with a fourth engaging hole 5123R.

A first rope portion 60a extending from a fourth engaging body 64 is guided from a right rope groove 5122R toward a lower side of a second outer rear pulley 324 to be hooked on a rear rope groove 35d. The first rope portion 60a guided toward a lower side of the second outer rear pulley 324 is further guided toward an upper end side of a second outer front pulley 314 to be hooked between a rear rope groove 35d and a front rope groove 34d in the shape of S character. Still further, the first rope portion 60a is guided toward a first forceps pulley 23 to be hooked on a first rope groove 23b, to reach a first engaging body 61.

As described in the foregoing, a loop-shaped rope 6 is hooked on a first forceps pulley 23 and a second forceps pulley 25. Then, a rope main body 60 is folded at a first forceps pulley 23, a second forceps pulley 25, a left driving pulley 512L and a right driving pulley 512R, with which a first to fourth engaging bodies 60a to 60d of the loop-shaped rope 6 are engaged.

At forceps 20, a first rope portion 60a and a second rope portion 60b are folded over a first engaging body 61 engaged with a first forceps pulley 23, and a third rope portion 60c and a fourth rope portion 60d are folded over a second engaging body 62 engaged with a second forceps pulley 25. At a first driving portion 51, the second rope portion 60b and the third rope portion 60c are folded over a third engaging body 63 engaged with a left driving pulley 512L, and the first rope portion 60a and the fourth rope portion 60d are folded over a fourth engaging body 64 engaged with a right driving pulley 512R.

As shown in FIG. 3A, when a right driving pulley 512R is rotated in a direction of an arrow A by driving a right driving motor, a tension is generated on a first rope portion 60a, so that a first forceps pulley 23 rotates clockwise and a first forceps piece main body 22 rotates in an opening direction. Here, a tension force is transmitted to a second rope portion 60b, so that a left driving pulley 512L also follows a rotation in a direction of the arrow A. Via the left driving pulley 512L, a second forceps pulley 25 rotates counterclockwise by a third rope portion 60c, and a second forceps piece main body 24 rotates in the opening direction. Still further, when a right driving pulley 512R is rotated by driving a right driving motor in a direction of an arrow B opposed to the arrow A, the first forceps piece main body 22 and the second forceps piece main body 24 rotate in a closing direction.

In the meantime, when a left driving pulley 512L is rotated in a direction of an arrow A by driving a left driving motor, a first forceps piece main body 22 and a second forceps piece main body 24 can also rotate in an opening direction. Still further, if motors freely rotatable when no current is supplied are used for driving motors on the right and left, driving pulleys smoothly follow the motors' rotation. Still further, it is also possible to provide a clutch between each driving pulley and each driving motor, in order to transmit clockwise and counterclockwise rotations from the driving motor to the driving pulley, but not to transmit these inversely, so as to allow a free rotation of the driving pulley.

A first rope portion 60a and a second rope portion 60b of a rope main body 60 are hooked to have inclination, on both sides of a first engaging body 61 engaged with a first engaging hole 23c of a first forceps pulley 23, on an upper end portion of a first outer front pulley 311 and on an upper end portion of a second outer front pulley 314. Therefore, when the first rope portion 60a and the second rope portion 60b are pulled backward, a holder 21 rotates upward about a P axis at a tip of an arm portion 4. In this case, a right driving pulley 512R is rotated in a direction of an arrow A, while a left driving pulley 512L in a direction of an arrow B at the same time. Here, a second forceps pulley 25 rotates upward about a P axis, so that a third rope portion 60c and a fourth rope portion 60d are tensioned without slackening.

On the contrary, when a third rope portion 60c and a fourth rope portion 60d are pulled backward at the same time, a holder 21 rotates downward about a P axis on a P supporting axis 26b at a tip of an arm portion 4. In this case, a right driving pulley 512R is rotated in a direction of an arrow B, while a left driving pulley 512L in a direction of an arrow A at the same time. Here, a second forceps pulley 25 rotates downward about a P axis, so that a first rope portion 60a and a second rope portion 60b are tensioned without slackening.

Next, reference is made to an operation of the left driving portion 51L and the right driving portion 51R along the forward and backward direction of the arm portion 4 to maintain the opened and closed state of the first forceps piece 20A and the second forceps piece 20B and to change the directions of the forceps 20 about the Y axis direction.

The second rope portion 60b and the third rope portion 60c are folded at the left driving pulley 512L of the left driving portion 51L to be arranged between the first forceps pulley 23 and the second forceps pulley 25. The first rope portion 60a and the fourth rope portion 60d are folded at the right driving pulley 512R of the right driving portion 51R to be arranged between the first forceps pulley 23 and the second forceps pulley 25.

Still further, a first rope portion 60a and a second rope portion 60b are arranged on both sides on the right and left over a first engaging body 61 engaged with a first engaging hole 23c of a first forceps pulley 23. In the same manner, a fourth rope portion 60d and a third rope portion 60c are arranged on both sides on the right and left over a second engaging body 62 engaged with a second engaging hole 25c of a second forceps pulley 25.

Therefore, when a left slider 511L is slid rearward from an arm portion 4, a second rope portion 60b and a third rope portion 60c are pulled backward by a left driving pulley 512L at the same time. In this manner, a counterclockwise rotation force is applied at the same time to a first forceps pulley 23 and a second forceps pulley 25 via a first engaging body 61 and a second engaging body 62. Here, when a right slider 511R is slid forward from the arm portion 4 in a synchronous manner with a slide of the left slider 511L, the first forceps pulley 23 and the second forceps pulley 25 rotate counterclockwise to a predetermined angle depending on a slide amount, to maintain an opening and closing angular position of a first forceps piece 22 and a second forceps piece 24 and to change a direction. On the contrary, when the right slider 511R is slid rearward from the arm portion 4 and the left slider 511L is slid forward from the arm portion 4, the first forceps pulley 23 and the second forceps pulley 25 rotate clockwise to a predetermined angle to maintain an opening and closing angular position of a first forceps piece 22 and a second forceps piece 24 and to change a direction.

A second driving portion 52 slides a left slider 511L and a right slider 511R in different directions along a forward and backward direction of an arm portion 4. The second driving portion 52 includes a slider driving pulley 521 being a fifth rotating body, a pulley holder 522 for rotatably holding the slider driving pulley 521, a motor axis 523 of a slider driving motor (not shown) for rotatably driving the slider driving pulley 521, and a holder fixing screw 524 for fixing the pulley holder 522 on a screw hole portion 44.

On a slider driving pulley 521, a motor axis 523 is inserted to an axial center of a pulley main body 521a, and an axial hole 521b to which the motor axis is coupled by a spline is formed; further, a rope groove 521c on which a rope main body 70 is hooked is formed on an outer circumference. A central engaging body 71 being a central fixing spot of a non-loop shaped wire rope 7 is engaged with an engaging hole 521d formed on a spot of the rope groove 521c, so that the non-loop shaped wire rope 7 is fixed to the slider driving pulley 521 in a tangential direction. The slider driving pulley 521 slidably drives a left slider 511L and a right slider 511R via the non-loop shaped wire rope 7.

On a pulley holder 522, an attaching frame portion 522a is formed on its front side, while a pulley housing portion 522b on its rear side. On the attaching frame portion 522a, a rear end portion of an arm portion 4 is inserted between its upper frame 522c and its lower frame 522d. On the upper frame 522c, a long hole 522e elongated in a forward and backward direction into which a holder fixing screw 524 is inserted is formed. On the lower frame 522d, an insertion groove (not shown) is formed along the forward and backward direction. On a lower surface of a rear end portion of the arm portion 4, a ridge 44a fitted into the insertion groove is formed along the forward and backward direction.

In a pulley housing portion 522b, a slider driving pulley 521 is disposed along a Y axis direction through an axial center of an axial hole 521b, and a motor axis 523 is non-rotatably coupled to the axial hole 521b. Both end portions of the motor axis 523 are pivotally supported on axial holes 522f respectively formed above and below the pulley housing portion 522b to be rotatable.

On a pulley holder 522, an attaching frame portion 522a is inserted into a rear end portion of an arm portion 4. Here, an insertion groove of a lower frame 522d is aligned with a ridge 44a of the arm portion 4 before insertion, then the pulley holder 522 is straightly inserted into the arm portion 4. The pulley holder 522 is fully inserted up to a predetermined position of the arm portion 4, and a holder fixing screw 524 is screwed into a screw hole portion 44, so that it is fixed to a rear end portion of the arm portion 4.

Now, a tension of a rope main body 60 of a loop-shaped rope 6 is smaller when a fixing position of a pulley holder 522 to a rear end portion of an arm portion 4 moves forward, while the tension is larger when the position moves rearward. Therefore, adjusting the fixing position of the pulley holder 522 makes it possible to adjust the tension of the rope main body 60 to a predetermined value.

When a slider driving motor as described above is driven to drive a motor axis 523 clockwise and to rotate a slider driving pulley 521 clockwise, a tensile force is generated on a rope main body 70 between a central engaging body 71 and a right terminal portion 73 of a non-loop shaped rope 7 to slidably move a right slider 511R rearward. Here, the rope main body 70 between the central engaging body 71 and a left terminal portion 72 is fed forward to slidably move forward from a left slider 511L. As a result, forceps 20 rotate clockwise about a Y axis to change direction.

On the contrary, when a slider driving motor as described above is driven to rotate a slider driving pulley 521 counterclockwise, a left slider 511L slidably moves rearward and a right slider 511R slidably moves forward, and forceps 20 are rotated counterclockwise about a Y axis to change direction.

As described in the foregoing, a single loop-shaped rope 6 is hooked on a first forceps pulley 23 and a second forceps pulley 25 of a first forceps piece 20A and a second forceps piece 20B constituting forceps 20 to enable an opening and closing operation as well as rotation movement in the Y axis and a P axis of the forceps 20. In this manner, it is enough to adjust a tension only of the single loop-shaped rope 6.

Shifting an attaching position of a pulley holder 522 of a second driving portion 52 in a forward and backward direction relative to an arm portion 4 makes it possible to adjust a tension of a single loop-shaped rope 6.

In this embodiment, it is possible to rotate forceps 20 about a Y axis by slidably moving a left slider 511L and a right slider 511R of a first driving portion 51 in opposite directions in a forward and backward direction of an arm portion 4, thus a single loop-shaped rope 6 enables three operations as described above.

Namely, instead of rotating pulleys by rotation of a loop-shaped rope 6 to obtain a rotation force about a Y axis, the loop-shaped rope 6 is divided into two loop portions by an engaging area between a first forceps pulley 23 and a second forceps pulley 25, a second rope portion 60b and a third rope portion 60c constituting a loop section on the left from an arm portion 4 are pulled by a left slider 511L, and a first rope portion 60a and a fourth rope portion 60d constituting a loop section on the right are pulled by a right slider 511R to realize a rotation of forceps 20 about a Y axis.

According to this embodiment, an opening and closing operation of forceps 20, their direction changing operation about a Y axis, and their direction changing operation about a P axis are all realized by a single loop-shaped rope 6. On this ground, an opening and closing operation of forceps 20 can be realized by rotating one of a left driving pulley 512L and a right driving pulley 512R of a first driving portion 51. Here, a mechanically balanced state is maintained for a tension applied on a first rope portion 60a to a fourth rope portion 60d of a loop-shaped rope 6, so only a direction of forceps 20 about the Y axis is changed, without changing their direction about the P axis. This also applies to an operation for changing the direction of the forceps 20 about the Y axis by synchronously driving the left driving pulley 512L and the right driving pulley 512R, as well as to an operation for changing the direction of the forceps 20 about the P axis by rotating a slider driving pulley 521 of a second driving portion 52 to slidably drive the left driving pulley 512L and the right driving pulley 512R.

Accordingly, when one of the operations is made to forceps 20, there is no need for driving control of other operations, so that it is possible to easily realize each of the operations to the forceps 20 by each driving motor of a driving portion 5 with high precision. Still further, it is possible to achieve an operation feeling with a good response.

### Embodiment 2

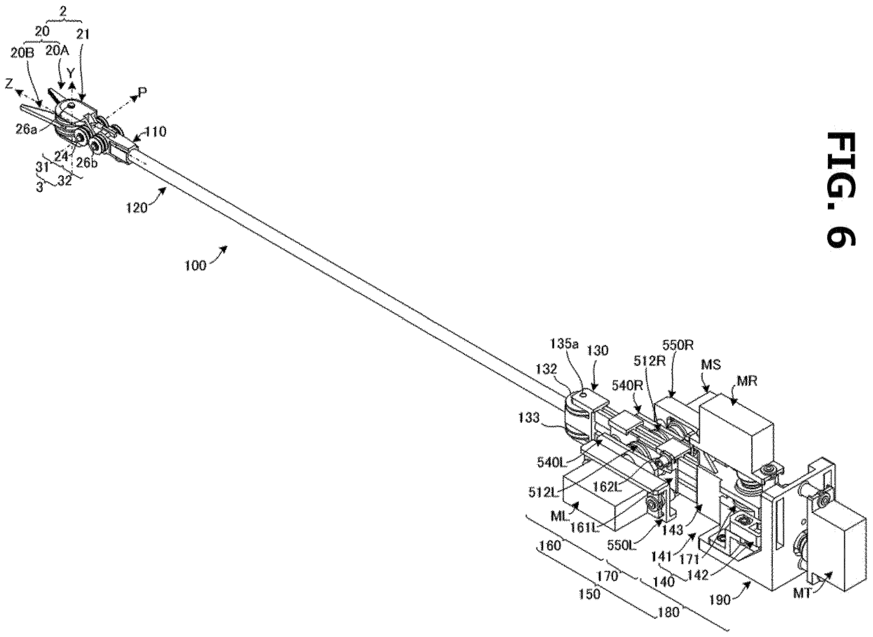

FIGS. 6 to 12 show Embodiment 2.

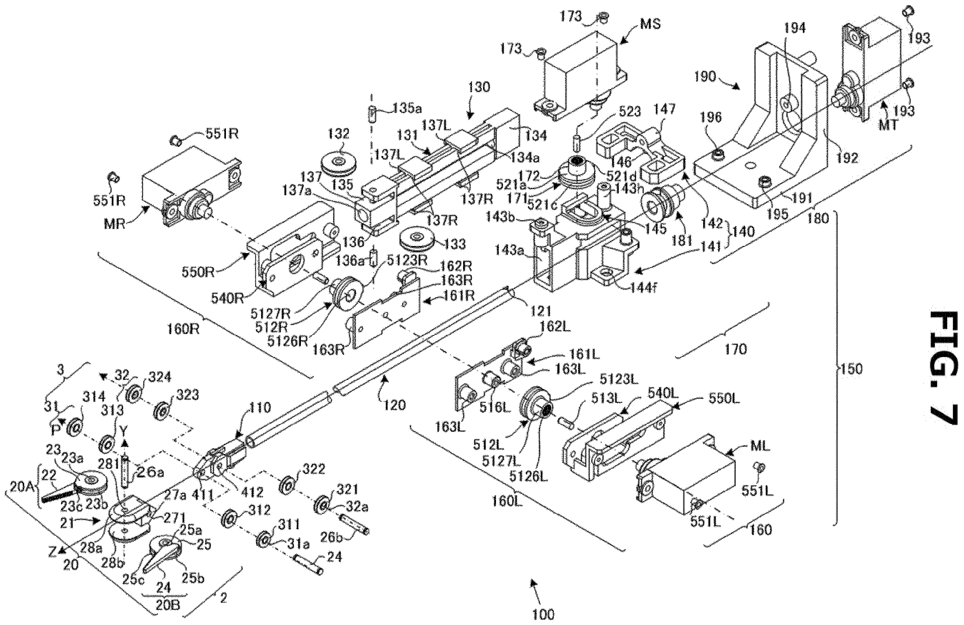

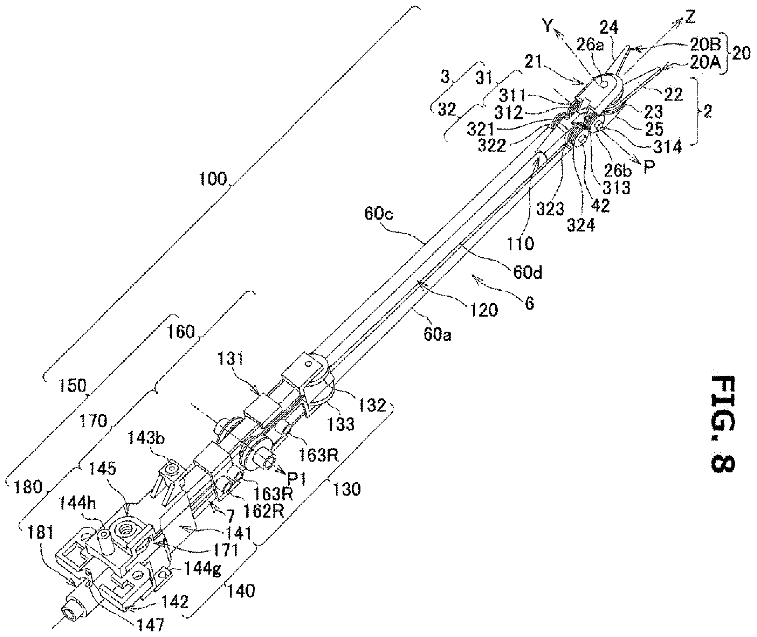

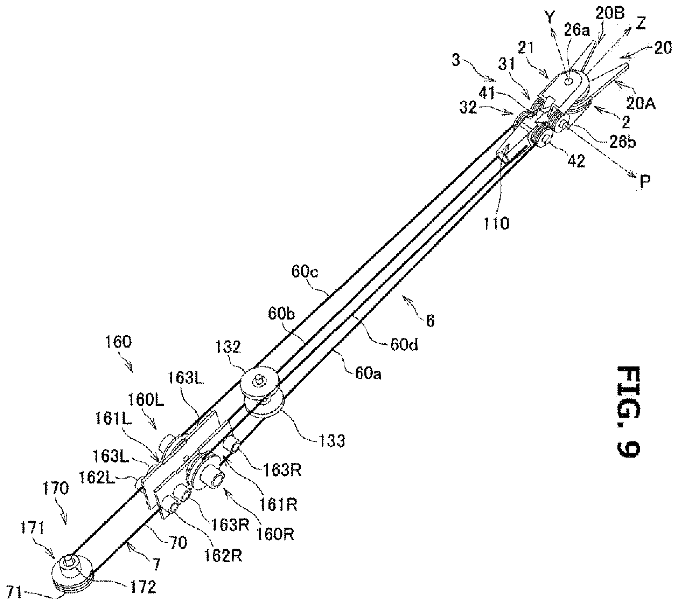

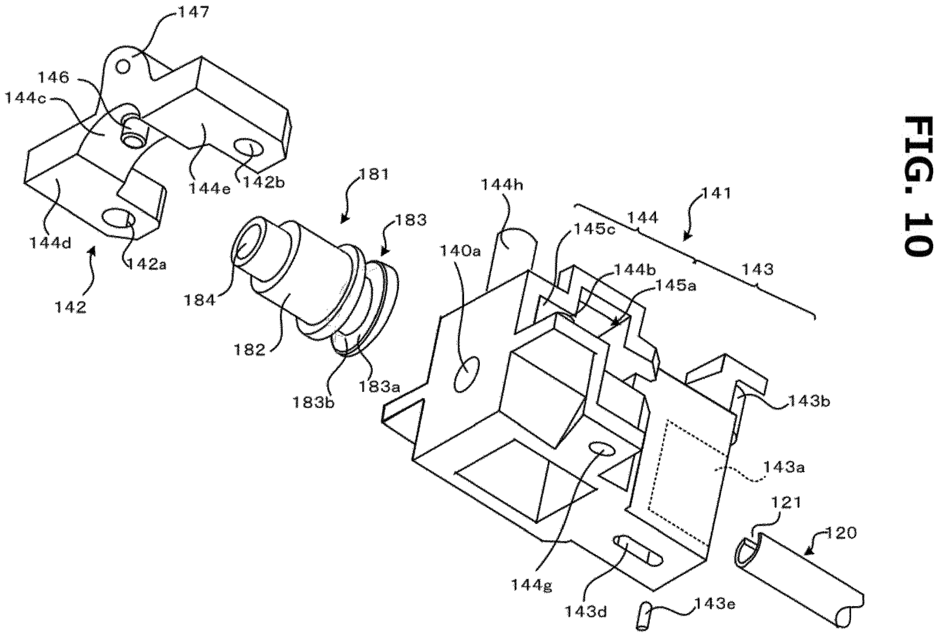

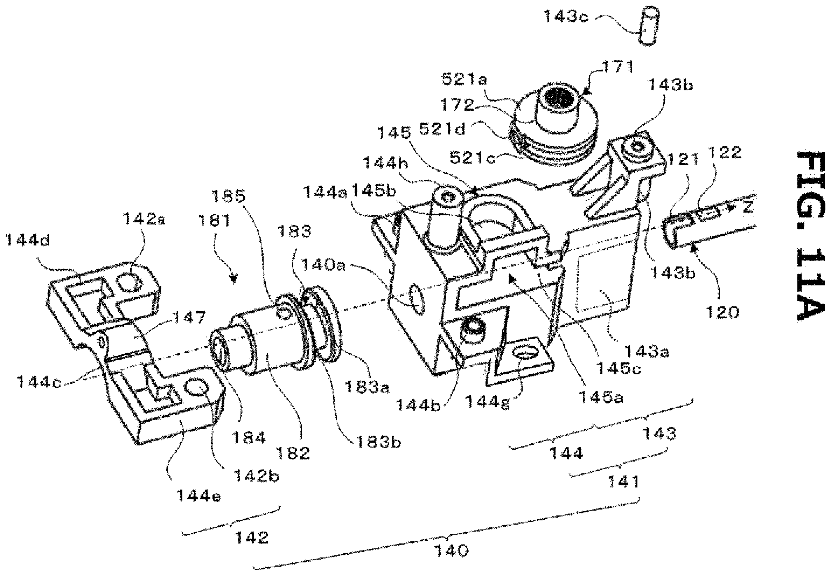

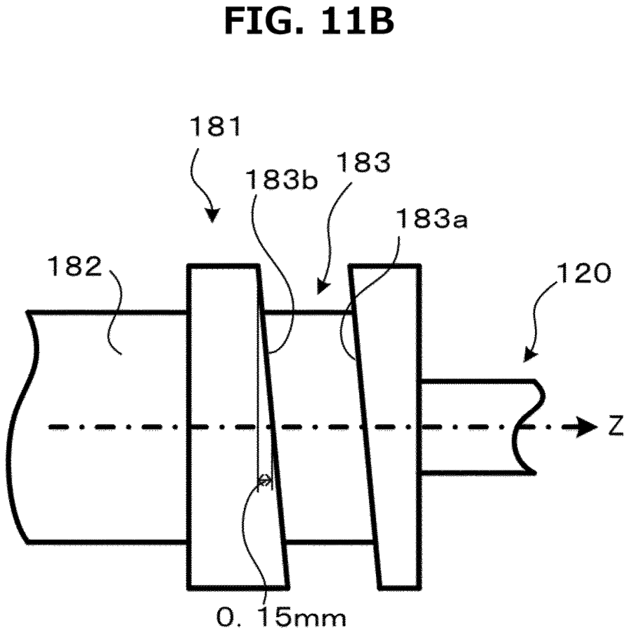

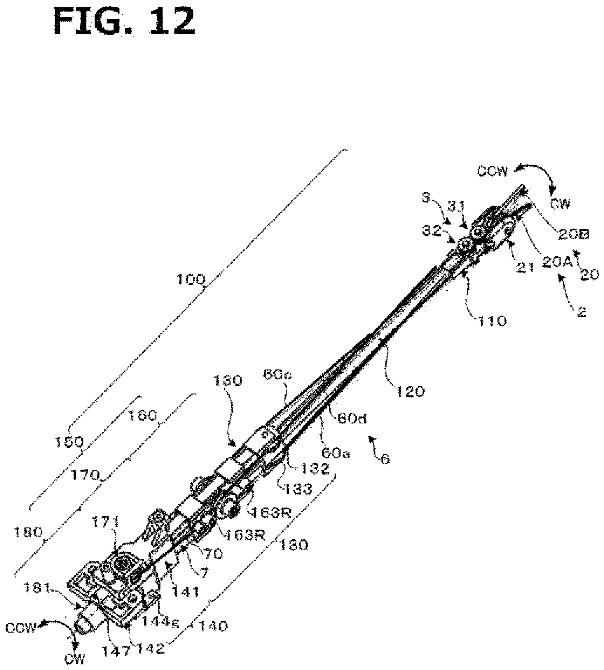

FIG. 6 shows a perspective view of appearance of a manipulator according to Embodiment 2 of the invention; FIG. 7 is an exploded perspective view of a manipulator as shown in FIG. 6; FIG. 8 is a perspective view illustrating an arranged state in a manipulator as shown in FIG. 6, in which a loop-shaped rope is hooked on pulleys; FIG. 9 is a perspective view illustrating an arranged state of a loop-shaped rope and non-loop-shaped rope in a manipulator as shown in FIG. 8; FIG. 10 is an exploded perspective view of a second driving portion and a third driving portion of a manipulator as shown in FIG. 6, as seen from below; FIG. 11A is an exploded perspective view of a second driving portion and a third driving portion of a manipulator as shown in FIG. 6, as seen from above; FIG. 11B is a view illustrating a cam surface of a cylindrical cam portion of a third driving portion as shown in FIG. 11A; and FIG. 12 is a view illustrating a state, in which a supporting pipe 120 of a manipulator as shown in FIG. 8 is operated by rotation by 90 degrees.

In Embodiment 1 as described above, an opening and closing operation as well as rotation movement about a Y axis and a P axis of the forceps 20 are all possible. In contrast, a manipulator 100 in Embodiment 2 enables, in addition to three operations as described above, rotations of an operating portion 2 together with a tip-side pulley portion 3 in a clockwise (CW) and counterclockwise (CCW) directions about a Z axis. In FIGS. 6 to 12, parts identical to those shown in FIGS. 1 to 5 are labeled with identical reference numerals and reference is not made to such parts.

### Overall Structure of Manipulator 100

A manipulator 100 includes a tip-attaching portion 110 to which an operating portion 2 and a tip-side pulley portion 3 are integrally attached; a supporting pipe 120 constituting a rotating supporting body being a manipulator main body; a first base portion 130 constituting a first fixing portion; a second base portion 140 constituting a second fixing portion; a driving portion 150; a loop-shaped rope 6; and a non-loop-shaped rope 7. The driving portion 150 includes a first driving portion 160 (corresponding to a first driving portion 51 in Embodiment 1), a second driving portion 170 (corresponding to a second driving portion 52 in Embodiment 1) and a third driving portion 180. In the meantime, the supporting pipe 120 and the tip-attaching portion 110 are here separate parts, but it is also possible to attach an operating portion 2 and a tip-side pulley portion 3 to a tip portion of the supporting pipe 120.

### Structure of Tip-Attaching Portion 110

A tip-attaching portion 110 is formed in the shape of prismatic column, having a structure identical to that of a tip portion of an arm portion 4 as shown in FIGS. 1 and 2. The tip-attaching portion 110 has an inserting tip portion 41 on its tip, to which a holder 21 provided with forceps 20 is attached to be rotatable about a P axis. Still further, a tip of a supporting pipe 120 is inserted into an insertion hole (not shown) formed on a rear end portion of the tip-attaching portion 110 to fix the tip-attaching portion 110 to the supporting pipe 120.

### Structure of Supporting Pipe 120

A supporting pipe 120 is formed of a round pipe, and a notch 121 for positioning and a fixing hole 122 for fixing are formed on its rear end portion.

### Structure of First Base Portion 130

A first base portion 130 and a second base portion 140 are fitted together, such that a supporting pipe 120 is freely rotatable and a fitting position is modifiable along a Z axis direction. The second base portion 140 is disposed rearward from the first base portion 130, and the first base portion 130 and the second base portion 140 are coupled to each other, such that their fixing position along a forward and backward direction is modifiable.

A first driving portion 160 of a driving portion 150 is provided on a first base portion 130. A second driving portion 170 and a third driving portion 180 of the driving portion 150 is provided on a second base portion 140.

A first base portion 130 includes a first main body portion 131 formed in the thin square shape along a Z axis direction, a first regulating pulley 132 and a second regulating pulley 133, both being regulating members provided on a front portion of the first main body portion 131. In the first main body portion 131, a fitting block 134 in the square shape provided on a rear end portion is fitted along the Z axis direction into a square fitting concave portion 143a formed on a front block of a second base portion 140. Accordingly, the first base portion 130 and the second base portion 140 are coupled to each other to be non-rotatable about a Z axis relative to each other. A supporting pipe 120 is inserted into a through hole 134a provided on the fitting block 134 to be rotatable and movable in an axial direction.

A first regulating pulley 132 and a second regulating pulley 133 are disposed to be separated from each other on a front portion of a first main body portion 131. The first regulating pulley 132 is disposed inside a first concave portion 135, while the second regulating pulley 133 in a second concave portion 136. A supporting pipe 120 is inserted into a through hole 137a provided on a separating wall 137 provided between the first concave portion 135 and the second concave portion 136 to be rotatable and movable in an axial direction. The first regulating pulley 132 is supported by a supporting axis 135a passing through the first concave portion 135 in an upward and downward direction to be freely rotatable, while the second regulating pulley 133 - by a supporting axis 136a passing through the second concave portion 136 in an upward and downward direction to be freely rotatable.

A left guide portion 137L and a right guide 137R facing each other in an upward and downward direction are respectively formed on both side portions on the right and left of a first main body portion 131. The left guide portion 137L and the right guide 137R are provided to be separated from each other along the upward and downward direction. A left slider 161L and a right slider 161R of a first driving portion 160 are respectively attached to the left guide portion 137L and the right guide 137R, which slidably hold the left slider 161L and the right slider 161R along a Z axis direction.

### Structure of First Driving Portion 160

A first driving portion 160 includes a left driving portion 160L provided on a left guide portion 137L (having a structure identical to a left driving portion 51L) and a right driving portion 160R provided on a right guide portion 137R (having a structure identical to a right driving portion 51R), wherein the both driving portions are formed symmetrically on the right and left.

In a left driving portion 160L, a left driving pulley 512L is rotatably supported by a pulley supporting axis 516L of a left slider 161L, and the left driving pulley 512L is rotatably driven by a motor axis 513L of a left driving motor ML. A rope locking projection 162L is formed on a rear end portion outside the left slider 161L to lock a left terminal portion 72 in the shape of ball provided on a left end of a rope main body 70 of non-loop-shaped rope 7. When the rope main body 70 is pulled backward, a left slider 161L is guided backward toward a left guide rail portion 137L to slide.

In a left slider 161L, a left holding plate 540L for holding a left driving pulley 512L is disposed outside the left driving pulley 512L along a forward and backward direction. The left holding plate 540L is fixed to the left slider 161L by screwing a screw (not shown) into a left screw hole boss 163L for fixing the plate. A left motor bracket 550L is attached to the outside of the left holding plate 540L. The left driving motor ML is fixed to the left motor bracket 550L by a screw 551L. A third rope portion 60c is hooked on a rope groove of a first regulating pulley 132 on the left side, while a second rope portion 60b is hooked on a rope groove of a second regulating pulley 133 on the left side. Even if the second rope portion 60b and the third rope portion 60c are twisted by a rotation of a supporting pipe 120, the first regulating pulley 132 and the second regulating pulley 133 regulate a movement of the second rope portion 60b and the third rope portion 60c in a Y axis direction to contact the supporting pipe 120.

A right driving portion 160R have the same structure as a left driving portion 160L, the identical elements or parts have identical reference numerals, but with ending R and without description on their structure.

As shown in FIG. 6, a loop-shaped rope 6 is hooked on a left driving pulley 512L, a right driving pulley 512R, a first forceps pulley 23 and a second forceps pulley 25, as well as around each pulley (311 to 314) of a front row pulley portion 31 and each pulley (321 to 324) of a rear row pulley portion 32 in the shape of S, as in Embodiment 1. A fourth rope portion 60d is hooked on a pulley groove of a first regulating pulley 132 on the right side, while a third rope portion 60c is hooked on the left side. Still further, a first rope portion 60a is hooked on a pulley groove of a second regulating pulley 133 on the right side, while a second rope portion 60b is hooked on the left side.

### Structure of Second Base Portion 140 and Second Driving Portion 170

A second base portion 140 includes a second main body portion 141 substantially in the cuboid shape and a cam holder 142. The second main body portion 141 includes a front block 143 provided on a front portion, a rear block 144 provided on a rear portion and an axial hole 140a running through a central portion in a Z axis direction. A supporting pipe 120 is inserted into an axial hole 140a to be rotatable and movable along a Z axis direction. A fitting block 134 of a first main body portion 131 is fitted into a square fitting concave portion 143a formed on the front block 143 to be non-rotatable about a Z axis. The fitting block 134 is fixed into the fitting concave portion 143a using fasteners (not shown) such as screws. A fitting position the fitting block 134 to the fitting concave portion 143a in a Z axis direction can be adjusted by loosening the fasteners. Screw hole bosses 143b, 144h for fixing a motor are provided on upper surfaces of the front block 143 and the rear block 144. A slider driving motor MS of a second driving portion 170 is fixed to the screw hole bosses 143b, 144h for fixing the motor by screws 173.

As shown in FIG. 10, an elongated guide hole 143d is formed in a Z axis direction on a bottom wall portion of the front block 143, and a guide pin 143e provided on a base 190 as shown in FIG. 7 is engaged with the guide hole 143d. A second base portion 140 integrated with a first base portion 130 is regulated in its rotation about a Z axis by engaging the guide pin 143e with the guide hole 143d.

A pulley holder 145 for rotatably holding a slider driving pulley 171 of a second driving portion 170 is formed on an upper portion of a rear block 144. The pulley holder 145 includes an opening portion 145a, wherein a right side end surface has the shape matching with a central longitudinal cross section shape of the slider driving pulley 171, a bearing portion 145b for supporting a central axial portion 172 of the slider driving pulley 171 and a concave portion 145c into which a pulley main body 521a of the slider driving pulley 171 is inserted.

As shown in FIG. 11A, engaging projections 144a, 144b are formed on rear end portions on the right and left of a rear block 144. The engaging projections 144a, 144b are engaged with engaging holes 142a, 142b on the right and left of a cam holder 142, and the cam holder 142 is fixed to a second base portion 140 at a predetermined position. Engaging holes 144f, 144g for fixing a base portion 140 to a base 190 shown in FIGS. 6, 7 are formed on the front side in a Z axis direction on the engaging projections 144a, 144b.

As shown in FIGS. 10, 11A, in a cam holder 142, engaging holes 142a, 142b are formed on front portions of overhanging portions 144d, 144e provided on the right and left of a bridge portion 144c formed in an arc shape. As shown in FIG. 10, a cam engaging axis 146 in a cylindrical shape is formed facing downward on a lower surface of the center in a right and left direction of the bridge portion 144c. The cam engaging axis 146 is engaged with a cam groove 183 of a cylindrical cam portion 181 of a third driving portion 180. A screw hole boss 147 for fixing the cam holder 142 to a base 190 is formed on an upper portion of the bridge portion 144c.

### Structure of Third Driving Portion 180

A third driving portion 180 includes a cylindrical cam portion 181, a rotational driving motor MT and a base 190. The base 190 is formed substantially in the shape of L by a horizontal base portion 191 and a vertical base portion 192. The rotational driving motor MT is fixed to the vertical base portion 192 by a screw 193. A screw (not shown) passing through a screw insertion hole 194 formed on the vertical base portion 192 is screwed into a screw hole boss 147 of a cam holder 142, and a second base portion 140 is fixed to the base 190 via the cam holder 142. A pair of engaging projections 195, 196 on the right and left are formed on the horizontal base portion 191. Engaging holes 144f, 144g of a rear block 144 are engaged with the engaging projections 195, 196 on the right and left, and a base portion 140 is positioned on the base 190. The cylindrical cam portion 181 includes a main body portion 182 formed in a cylindrical shape, and a cam groove 183 formed on a front portion of the main body portion 182 along a circumferential direction. An axial hole 184 is formed along a Z axis direction to pass through a rotation center of the main body portion 182. A rear portion of the axial hole 184 is formed on a spline groove (not shown) coupled e.g. by spline to an output axis of the rotational driving motor MT. Therefore, when the rotational driving motor MT is driven, the cylindrical cam portion 181 is rotated about a Z axis.

On the other hand, a rear end portion of a supporting pipe 120 is inserted into a front portion of an axial hole 184. A positioning key (not shown) is provided inside the axial hole 184. The supporting pipe 120 is positioned rearward along a Z axis direction with a notch engaging in the axial hole 184, as well as about a Z axis. A screw hole 185 reaching the axial hole 184 is formed in a radial direction. In a state in which the supporting pipe 120 is positioned by the positioning key, a supporting pipe fixing screw (not shown) is screwed into a screw hole 185 to engage a screw end portion of the supporting pipe fixing screw with a fixing hole 122 of the supporting pipe 120. Therefore, the supporting pipe 120 is integrally fixed to a cylindrical cam portion 181.

A cam groove 183 abuts against a cam engaging axis 146, with a front side wall surface 183a and a rear side wall surface 183 provided on opposite sides in a forward and backward direction of the cam groove 183 being cam surfaces. Reference is made to a cam locus of the cam surfaces formed of the front side wall surface 183a and the rear side wall surface 183b, wherein a position at which a notch 121 of a supporting pipe 120 is positioned at 90 degrees on a Y axis is defined as a rotation angle of 0 degree, with a Y, P and Z axes being three axes perpendicular to each other.

When a cylindrical cam surface 181 is rotated for example in a CW direction and CCW direction to 90 degrees with reference to a rotation angle of 0 degree about a Z axis, a cam engaging axis 146 abuts against a rear side wall surface 183b of a cam groove 183, so that the cylindrical cam surface 181 moves backward along a Z axis direction. When the cylindrical cam surface 181 is rotated from any rotation angle position toward the rotation angle of 0 degree being a reference position, the cam engaging axis 146 abuts against a front side wall surface 183a of the cam groove 183, so that the cylindrical cam surface 181 moves forward along the Z axis direction.

When a cylindrical cam surface 181 is rotated for example in a CW direction and CCW direction from a reference position of 0 degree about a Z axis, due to engagement between a cam groove 183 and a cam engaging axis 146, the cylindrical cam surface 181 moves backward in a Z axis direction following a cam locus with rotation. In this manner, a supporting pipe 120 moves backward in the Z axis direction with rotation. When the cylindrical cam surface 181 is rotated from any rotation angle position toward a position of the rotation angle of 0 degree, the supporting pipe 120 moves forward in the Z axis direction with rotation.

In this embodiment, a cam locus of a cam groove 183 is set such that cam surfaces (a front side wall surface 183a and a rear side wall surface 183b of the cam groove 183) are located forward in the Z axis direction, as the cam groove travels toward 0 degree and 180 degrees from a cam top position of 90 degrees, where the cam top position of the cam groove 183 is defined as a position of 90 degrees in a Y axis perpendicular to a P supporting axis 26b (a rear supporting axis 42).

FIGS. 8, 9 show a state in which forceps 20 are held at a reference position as described above; a first rope portion 60a and a fourth rope portion 60d of a loop-shaped rope 6 are located on the right side of a supporting pipe 120, while a second rope portion 60b and a third rope portion 60c on the left side of the supporting pipe 120. In a state in which the supporting pipe 120 stops at a reference position as described above, the second rope portion 60b and the third rope portion 60c, as well as the first rope portion 60a and the fourth rope portion 60d of the loop-shaped rope 6 all extend straight relative to the supporting pipe 120, without being twisted around the supporting pipe 120.

In this embodiment, when a left driving pulley 512L or a right driving pulley 512R of a first driving portion 160 is rotated, a first forceps pulley 23 and a second forceps pulley 25 rotate relative to each other about a Y axis to enable an opening and closing operation of forceps 20. When the left driving pulley 512L and the right driving pulley 512R are synchronously rotated, the forceps 20 rotate about the Y axis to enable an operation for changing directions of the forceps 20.

When a slider driving pulley 171 of a second driving portion 170 is rotated in a CW and CCW directions, a left slider 161L or a right slider 161R of a first driving portion 160 is guided by a left guide portion 137L and a right guide 137R provided on both side portions on the right and left of a first main body portion 131 to slide forward and backward along a Z axis direction, and to rotate a holder 21 in a CW and CCW directions about a P axis; in this manner, an operation for changing directions about the P axis relative to forceps 20 is performed.

When a cylindrical cam portion 181 of a third driving portion 180 is rotated in a CW and CCW directions about a Z axis, a supporting pipe 120 rotates in a CW and CCW directions and thus moves forward and backward along a Z axis direction. As shown in FIG. 12, when the supporting pipe 120 rotates e.g. in a CCW direction about a Z axis, a tip-side pulley portion 3 provided on a tip-attaching portion 110, a second rope portion 60b, a third rope portion 60c arranged between a left driving pulley 512L and a right driving pulley 512R, a first rope portion 60a and a fourth rope portion 60d are all twisted around the supporting pipe 120. When the first rope portion 60a to the fourth rope portion 60d are all twisted, a tension on the loop-shaped rope 6 increases. When the tension on the loop-shaped rope 6 increases, a contact pressure between each pulley and the loop-shaped rope 6 increases, which may lead to a deterioration in quality of operation feelings in operation for changing directions of forceps 20 about a Y axis, an opening and closing operation of the forceps 20 and operation for changing their directions about a P axis.

However, in this embodiment, as a supporting pipe 120 rotates in a CW and CCW directions from a reference position, a cam groove 183 of a cylindrical cam portion 181 abut against a cam engaging axis 146, so that the cylindrical cam portion 181 goes back along a Z axis direction to shorten respective distances between a tip-side pulley portion 3, a left driving pulley 512L and a right driving pulley 512R, in order to cancel an increase in tension on the loop-shaped rope 6.

Here, in a state in which a left driving pulley 512L and a right driving pulley 512R are disposed at an identical position in a Z axis direction, a central axis line of the left driving pulley 512L and the right driving pulley 512R (also referred to as driving pulley axis line) is defined as P1. When a supporting pipe 120 rotates 90 degrees, with an axial distance being 142 mm between a P supporting axis 26b of a tip-side pulley portion 3 and a driving pulley axis line PI in the Z axis direction, a variation of each length of each of a first rope portion 60a to a fourth rope portion 60d is 0.15 mm. Therefore, a cam locus of a cam groove 183 of a cylindrical cam portion 181 should be set such that the cylindrical cam portion 181 goes back 0.15 mm along a Z axis direction, when the cylindrical cam portion 181 is rotated to 90 degrees in a CW and CCW directions from the reference position.

In this manner, due to a rotation of a cylindrical cam portion 181, a supporting pipe 120 moves forward and backward, so that it is possible to maintain a tension on a first rope portion 60a to a fourth rope portion 60d to a constant value, regardless of rotation angle of the supporting pipe 120. Therefore, even if a direction of forceps 20 is changed due to a rotation of the supporting pipe 120, it is possible to perform an opening and closing operation of the forceps 20, operation for changing their directions about a Y axis, as well as about a P axis without discomfort.

Moreover, an opening and closing position of forceps 20, their angles about a Y axis as well as about a P axis do not change, as accompanied by a rotation of a supporting pipe 120 by driving a third driving portion 180. Therefore, it is not necessary to correct the opening and closing position of the forceps 20, their angles about the Y axis as well as about the P axis by driving motors ML, MR of a first driving portion 160 and a second driving portion 170, in order to maintain the opening and closing position of the forceps 20, their angles about the Y axis as well as about the P axis.

Namely, when a first rope portion 60a to a fourth rope portion 60d of a loop-shaped rope 6 are twisted around a supporting pipe 120 by a rotation of a supporting pipe 120, a first rope portion 60a to a fourth rope portion 60d are pulled between a first engaging body 61 to a fourth engaging body 64. This prevents an opening and closing of forceps 20, change in direction about a Y axis, as well as about a P axis.

Still further, driving motors ML, MR of a first driving portion 160 are attached to a first base portion 130 via motor brackets 550L, 550R and a left holding plate 540L and a right holding plate 540. A driving motor MS of a second driving portion 170 is directly attached to a second base portion 140. A driving motor MT of a third driving portion 180 is directly attached to a base 190. A first base portion 130 and a second base portion 140 are coupled to each other to be non-rotatable about a Z axis and non-movable along a Z axis direction. The second base portion 140 is positioned to the base 190 to be fixed thereto. This prevents a swivel of respective driving motors ML, MR, MS, MT of a first driving portion 160 to a third driving portion 180 about a Z axis, in rotating a supporting pipe 120 about the Z axis.

Therefore, no large inertial force is applied on a supporting pipe 120 during rotation, which causes no delay at start and no overrun at stop, and enables forceps 20 to rotate with high precision

Still further, in this embodiment, a fixing position of a first base portion 130 to a second base portion 140 is changed along a forward and backward direction, so that just a change in position of a left slider 161L and a right slider 161R along a Z axis direction enables a tension of a loop-shaped rope 6 to be adjusted.

### Industrial Applicability

A manipulator according to the invention can be used in the field of medicine e.g. for surgery and in the industrial field e.g. for industrial robot.

**Reference Sign List**

| | | | | |
|---|---|---|---|---|
| 1 | manipulator | | | |
| 2 | operation portion | | | |
| | 20 | forceps | 21 | holder |
| | 20A | first forceps piece | 20B | second forceps piece |
| | 22 | first forceps piece main body | | |
| | 23 | first forceps pulley | | |
| | 23a | first axial hole | 23b | first rope groove |
| | 23c | first engaging hole | | |
| | 23d | partition wall | 23e, 25e | communication groove |
| | 24 | second forceps piece main body | | |
| | 25 | second forceps pulley | | |
| | 25a | second axial hole | 25b | second rope groove |
| | 25c | second engaging hole | 25d | partition wall |
| | 26a | Y supporting axis | 26b | P supporting axis |
| | 27a, 27b | supporting bracket | 28a, 28b | supporting plate |
| | 271 | axial hole | 281 | axial hole |
| 3 | tip-side pulley portion | | | |
| | 31 | front row pulley portion | | |
| | 31a | front pulley axial hole | 34a - 34d | front rope groove |
| | 311 | first outer front pulley | 312 | first inner front pulley |
| | 313 | second inner front pulley | 314 | second outer front pulley |
| | 32 | rear row pulley portion | | |
| | 32a | respective axial hole | 35a - 35d | Rear rope groove |
| | 321 | first outer rear pulley | 322 | first inner rear pulley |
| | 323 | second inner rear pulley | 324 | second outer rear pulley |
| 4 | arm portion | | | |
| | 41 | inserting tip portion | 411 | front axial hole |
| | 42 | rear supporting axes | 412 | rear axial hole |
| | 43 | driving portion attaching portion | 44 | screw hole portion |
| | 44a | ridge | | |
| | 45 | left guide rail portion | 451 | left rail groove |
| | 46 | right guide rail portion | 461 | right rail groove |
| 5 | driving portion | | | |
| | 51 | first driving portion | | |
| | 51L | left driving portion | 51R | right driving portion |
| | 511L | left slider | 511R | right slider |
| | 512L | left driving pulley | | |
| | 5121L | left pulley main body | 5122L | left rope groove |
| | 5123L | third engaging hole | 5126L | left bearing hole |
| | 5127L | left pulley driving axis | | |
| | 513L | motor axis | 514Lleft | slider main body |
| | 515L | engaging claw portion | 516L | pulley supporting axis |
| | 517L | left rope fixing piece | 517R | right rope fixing piece |
| | 517a | opening portion | | |
| | 517b | slit | | |
| | 512R | right driving pulley | | |
| | 5121R | right pulley main body | 5122R | right rope groove |
| | 5123R | fourth engaging hole | | |
| | 513R | motor axis | | |
| | 514R | right slider main body | 515R | engaging claw portion |
| | 516R | pulley supporting axis | | |
| | 52 | second driving portion | | |
| | 521, 171 | slider driving pulley | | |
| | 521a | pulley main body | 521b | axial hole |
| | 521c | rope groove | 521d | engaging hole |
| | 522 | pulley holder | | |
| | 522a | attaching frame portion | 522b | pulley housing portion |
| | 522c | upper frame | 522d | lower frame |
| | 522e | long hole | 522f | axial holes |
| | 523 | motor axis | 524 | holder fixing screw |
| 6 | | | | |
| | 60loop-shaped wire rope | loop-shaped rope main body | | |
| | 60a | first rope portion | 60b | second rope portion |
| | 60c | third rope portion | 60d | fourth rope portion |
| | 61 | first engaging body | 62 | second engaging body |
| | 63 | third engaging body | 64 | fourth engaging body |
| | 65 | closing terminal portion | | |
| 7 | non-loop shaped wire rope | | | |
| | 70 | wire rope main body | 71 | central engaging body |
| | 72 | left terminal portion | 73 | right terminal portion |
| | 100 | manipulator | | |
| | 110 | tip-attaching portion | | |
| | 120 | supporting pipe | | |
| | 121 | notch | 122 | fixing hole |
| | 130 | first base portion | | |
| | 131 | first main body portion | 132 | first regulating pulley |
| | 133 | second regulating pulley | 134 | fitting block |
| | 135 | first concave portion | 135a | supporting axis |
| | 136 | second concave portion | 136a | supporting axis |
| | 137 | separating wall | | |
| | 137L | left guide portion | 137R | right guide |
| | 140 | second base portion | | |
| | 140a | axial hole | | |
| | 141 | second main body portion | | |
| | 142 | cam holder | | |
| | 142a, 142b | engaging hole | | |
| | 143 | front block | | |
| | 143a | fitting concave portion | 143b | screw hole boss |
| | 143c | screw | 143d | guide hole |
| | 144 | rear block | | |
| | 144a, 144b | engaging projection | 144c | bridge portion |
| | 144d, 144e | overhanging portion | 144f, 144g | engaging hole |
| | 144h | screw hole boss | | |
| | 145 | pulley holder | | |
| | 145a | opening portion | 145b | bearing portion |
| | 145c | concave portion | | |
| | 146 | cam engaging axis | 147 | screw hole boss |
| | 150 | driving portion | | |
| | 160 | first driving portion | | |
| | 160L | left driving portion | 160R | right driving portion |
| | 161L | left slider | 161R | right slider |
| | 162L | rope locking projection | 163L | left screw hole boss |
| | 170 | second driving portion | 172 | axial portion 173 screw |
| | 180 | third driving portion | | |
| | 181 | cylindrical cam portion | MT | rotational driving motor |
| | 182 | main body portion | 183 | cam groove |
| | 183a | front side wall surface | 183b | rear side wall surface |
| | 184 | axial hole | 185 | screw hole |
| | 190 | base | | |
| | 191 | horizontal base portion | 192 | vertical base portion |
| | 194 | screw insertion hole | 195, 196 | engaging projection |
| | ML | left driving motor | MR | right driving motor |
| | MS | slider driving motor | | |

## Claims

1. A manipulator comprising:
a manipulator main body extending along a main axis direction;
an operation portion in which a first operation piece having a first rotating body and a second operation piece having a second rotating body are rotatably provided on a first axis along an axis direction, the operation portion being attached to a front edge portion of the manipulator main body so as to be rotatable about a second axis perpendicular to a main axis, along with rotation of the first rotating body and the second rotating body in mutually different rotation directions, an opening and closing operation of the first operation piece and the second operation piece thereby being performed;
a left driving portion provided with a third rotating body and a right driving portion provided with a fourth rotating body, both of the driving portions being disposed on the right and left of a rear end portion of the manipulator main body and movable along the main axis direction;
a loop-shaped wire rope formed from an endless loop-shaped wire rope main body folded by each of the first to the fourth rotating bodies at four fixing spots sequentially from a first fixing spot, a second, a third and a fourth fixing spots at intervals from one another, the first fixing spot being fixed to the first rotating body, the second fixing spot being fixed to the second rotating body, the third fixing spot being fixed to the third rotating body, and the fourth fixing spot being fixed to the fourth rotating body;
a fifth rotating body provided on a rear end portion of the loop-shaped wire rope main body with a position of the fifth rotating body being variable along the main axis direction, the fifth rotating body being rotatable; and
a non-loop-shaped wire rope folded at the fifth rotating body, one end of the wire rope being fixed to the left driving portion, the other end to the right driving portion, and a central fixing spot of the wire rope to the fifth rotating body.

2. A manipulator comprising:
a manipulator main body extending along a main axis direction;
an operation portion in which a first operation piece having a first rotating body and a second operation piece having a second rotating body are rotatably provided on a first axis along a axis direction, the operation portion being attached to a front edge portion of the manipulator main body so as to be rotatable about a second axis perpendicular to a main axis, along with rotation of the first rotating body and the second rotating body in mutually different rotation directions, an opening and closing operation of the first operation piece and the second operation piece being thereby performed;
a first base portion disposed on a rear end portion of the manipulator main body to be relatively rotatable to the manipulator main body;
a second base portion disposed rearward of the manipulator main body with a fixed position of the second base portion to the first base portion being variable, and being relatively rotatable to the manipulator main body;
a left driving portion provided with a third rotating body and a right driving portion provided with a fourth rotating body, both of the driving portions being disposed on the right and left of a rear end portion of the first base and movable along the main axis direction;
a loop-shaped wire rope formed from an endless loop-shaped wire rope main body folded by each of the first to the fourth rotating bodies at four fixing spots sequentially from a first fixing spot, a second, a third and a fourth fixing spots at intervals from one another, the first fixing spot being fixed to the first rotating body, the second fixing spot being fixed to the second rotating body, the third fixing spot being fixed to the third rotating body, and the fourth fixing spot being fixed to the fourth rotating body;
a fifth rotating body rotatably provided on the second base portion;
a non-loop-shaped wire rope folded at the fifth rotating body, wherein one end of the wire rope is fixed to the left driving portion, the other end is fixed to the right driving portion, and a central fixing spot of the wire rope is fixed to its fifth rotating body; and
a cam portion fixed to the manipulator main body to make the manipulator main body movable along the main axis direction following a rotation about the main axis.

3. The manipulator according to one of claims 1 and 2, further comprising:
a tip-side rotating body portion provided on the front edge portion of the manipulator main body, the tip-side rotating body portion for hooking two rope portions of the loop-shaped wire rope as folded at the first rotating body and other two rope portions of the loop-shaped wire rope as folded at the second rotating body on the respective rotating bodies in the shape of letter S to guide four of the rope portions to the third rotating body and the fourth rotating body,
wherein the third rotating body and the fourth rotating body have a rotation axis parallel to the second axis.

4. The manipulator according to one of claims 2 and 3, further comprising:
regulating members disposed on a front portion of the first base portion for abutting against four rope portions respectively folded at the third rotating body and the fourth rotating body to regulate a movement of each of the rope portions along a second axis.

5. The manipulator according to any one of claims 1 to 4, wherein, each fixing spot provided on the manipulator main body is an engaging body engaged with an engaging hole provided on each rotating body of the first rotating body to the fourth rotating body.
